# EUROPÄISCHE PATENTSCHRIFT

(11) **EP 2 880 013 B1**
(45) Veröffentlichungstag und Bekanntmachung des Hinweises auf die Patenterteilung: **09.05.2018**
(21) Anmeldenummer: 13742231.7
(22) Anmeldetag: 29.07.2013
(51) Int. Cl.: C07C 231/12, C07C 233/15

(54) **VERFAHREN ZUM HERSTELLEN VON SUBSTITUIERTEN BIPHENYLEN DURCH C-H-AKTIVIERUNG**
METHOD FOR PRODUCING SUBSTITUTED BIPHENYLS USING C-H ACTIVATION
PROCÉDÉ DE FABRICATION DE BIPHÉNYLES SUBSTITUÉS PAR ACTIVATION C-H

(30) Priorität: 02.08.2012 EP 12179058
(43) Veröffentlichungstag der Anmeldung: 10.06.2015
(73) Patentinhaber: Bayer CropScience AG, 40789 Monheim am Rhein (DE)
(72) Erfinder: RIEDRICH, Matthias, 50735 Köln (DE); RODEFELD, Lars, 51375 Leverkusen (DE); VOLZ, Frank, 50825 Köln (DE)
(74) Vertreter: BIP Patents
(86) Internationale Anmeldenummer: PCT/EP2013/065925
(87) Internationale Veröffentlichungsnummer: WO 2014/019995

(56) Entgegenhaltungen:
- GORDON BRASCHE ET AL: "Twofold C-H Functionalization: Palladium-Catalyzed Ortho Arylation of Anilides", ORGANIC LETTERS, Bd. 10, Nr. 11, 1. Juni 2008 (2008-06-01), Seiten 2207-2210, XP055047254, ISSN: 1523-7060, DOI: 10.1021/ol800619c in der Anmeldung erwähnt
- JOANNA WENCEL-DELORD ET AL: "[RhIIICp*]-Catalyzed Dehydrogenative Aryl-Aryl Bond Formation", ANGEWANDTE CHEMIE INTERNATIONAL EDITION, Bd. 51, Nr. 9, 27. Februar 2012 (2012-02-27), Seiten 2247-2251, XP055047251, ISSN: 1433-7851, DOI: 10.1002/anie.201107842 in der Anmeldung erwähnt
- BI-JIE LI ET AL: "Multiple C-H Activations To Construct Biologically Active Molecules in a Process Completely Free of Organohalogen and Organometallic Components", ANGEWANDTE CHEMIE INTERNATIONAL EDITION, Bd. 47, Nr. 6, 25. Januar 2008 (2008-01-25), Seiten 1115-1118, XP055047422, ISSN: 1433-7851, DOI: 10.1002/anie.200704092
- SHABASHOV DMITRY ET AL: "Palladium-Catalyzed Anilide ortho-Arylation and Subsequent One-Pot Cyclization to Phenanthridines", THE JOURNAL OF ORGANIC CHEMISTRY, AMERICAN CHEMICAL SOCIETY ETC.|, vol. 72, no. 20, 1 January 2007 (2007-01-01), pages 7720-7725, XP002527991, ISSN: 0022-3263, DOI: 10.1021/JO701387M [retrieved on 2007-09-07]

## Beschreibung

Die vorliegende Erfindung betrifft ein neues Verfahren zum Herstellen von substituierten Biphenylen der Formel (III) durch Umsetzen von Arenen der Formel (I) mit Arenen der Formel (II) in Gegenwart eines Übergangsmetallkatalysators und mindestens eines Oxidationsmittels.

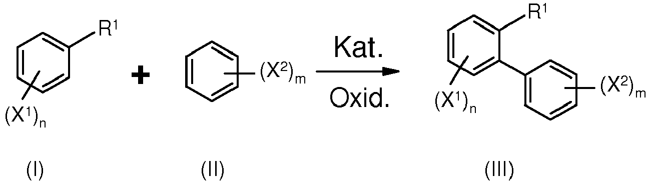

Biarylverbindungen, insbesondere Biphenylverbindungen, haben technische Bedeutung als Feinchemikalien, Zwischenprodukte für Pharmazeutika, optische Aufheller und Agrochemikalien.

Es ist bekannt, dass substituierte Biphenyle durch Suzuki-Kupplungen zugänglich sind. Hierfür werden allerdings teure Borin-/Boronsäuren mit Halogenarenen als Ausgangsverbindungen benötigt, die in Gegenwart eines Übergangsmetallkatalysators miteinander verknüpft werden, vgl. WO 2011/023324 A1. In letzter Zeit hat sich die Synthese von Biphenylen durch direkte zweifache C-H-Aktivierung als attraktive Alternative zu den bestehenden Syntheseverfahren (z.B. Suzuki-Reaktion) entwickelt. Hierbei werden Arene, die eine dirigierende Gruppe tragen, mit Arenen in Gegenwart von Übergangsmetallkatalysatoren und/oder geeigneten Aktivierungsreagenzien umgesetzt. Übersichtsartikel zu dieser Thematik findet man beispielsweise in Charles S. Yeung, Vy M. Dong, Chem. Rev. 2011, 111, 1215-1292. Eine Übersicht zur Pd-katalysierten Aryl-Aryl-Kupplung durch doppelte C-H-Aktivierung liefert Shu-Li You and Ji-Bao Xia, Top. Curr. Chem. 2010, 292, 165-194. Eine Übersicht über oxidative Kreuzkupplungen von Arenen findet man in James A. Ashenhurst, Chem. Soc. Rev. 2010, 39, 540-548. Der Vorteil der direkten zweifachen C-H-Aktivierung ist vor allem die gegenüber den bestehenden Syntheseverfahren (z.B. Suzuki-Reaktion) gesteigerte Nachhaltigkeit. Es werden keine präfunktionalisierten Kupplungspartner benötigt, wodurch die Zahl der Reaktionsschritte und der Abfall verringert werden.

Allerdings gibt es bei diesem Reaktionstyp auch eine Vielzahl von Schwierigkeiten, z.B. ungünstige Thermodynamik, die allgemein geringe Reaktivität von C-H Bindungen und Selektivitätsprobleme (Funktionalisierung einer C-H Bindung in Gegenwart von anderen C-H Bindungen, und die Konkurrenz zwischen Hetero- und Homokupplung). Bei den im Stand der Technik beschriebenen Pd-katalysierten Aryl-Aryl-Kupplungen durch zweifache C-H-Aktivierung beschränken sich die Beschreibungen von beispielsweise Gordon Brasche, Jorge García-Fortanet, Stephen L. Buchwald in Org. Lett. 2008, 10(11), 2207-2210 auf die Umsetzung von Aniliden mit elektronenreichen Arenen (enthaltend elektronenschiebende Substituenten wie z.B. Me, OMe, etc). Neben dem Palladium-haltigen Katalysator kommen hierbei DMSO in Kombination mit reinem Sauerstoff sowie TFA in Kombination mit Na₂S₂O₈ als zusätzliche Aktivierungsreagenzien bzw. Oxidationsmittel zum Einsatz. Elektronenarme Aromaten (enthaltend elektronenziehende Substituenten wie z.B. F, CF₃, CHF₂, Cl, etc) gelten als zu wenig reaktiv und lassen sich daher unter diesen Reaktionsbedingungen nur sehr schlecht oder gar nicht mit Aniliden zur Reaktion bringen.

Bi-Jie Li, Shi-Liang Tian, Zhao Fang, und Zhang-lie Shi in Angew. Chem., 2008, 47, 1115-1118, offenbaren Verfahren mit mehrfacher C-H Aktivierung zur Herstellung biologisch aktiver Moleküle, wobei das Verfahren frei von Organohalogen und Organometall-Verbindungen ist. Es wird beispielsweise ein Verfahren zur Palladium-katalysierten ortho-Arylierung von Acetanilid mit ortho-Xylol unter Verwendung von O₂ als Oxidationsmittel und PrOH als Lösungsmittel offenbart (Tab. 1, Nr. 6). Auch die Kupplung eines elektronenarmen Arenes (Fluorbenzol) mit N-Acetyltetrahydroquinolin wird beschrieben (Tab. 2, Nr. 8). Allerdings wurde hierfür eine wesentlich höhere Katalysatormenge benötigt, die Reaktion war nicht stereoselektiv und die Ausbeute betrug nur 48%.

Joanna Wencel-Delord, Corinna Nimphius, Frederic W. Patureau und Frank Glorius beschreiben in Angew. Chem. Int. Ed. 2012, 51, 2247-2251 eine Rh-katalysierte dehydrogenierende Aryl-Aryl-Kupplung, die den Einsatz elektronenarmer Aromaten erlaubt. Bisher wurden diese Umsetzungen durch C-H-Aktivierung allerdings nur mit Benzamiden als dirigierendem Kupplungspartner durchgeführt. Als Additive bzw. Oxidationsmittel wurden hier PivOH und CsOPiv sowie AgSbF₆ und Cu(OAc)₂ verwendet. Der Einsatz von Aniliden in der Umsetzung mit elektronenarmen Arenen (enthaltend elektronenziehende Substituenten wie z.B. F, CF₃, CHF₂, Cl, etc) konnte unter den im Stand der Technik vorbeschriebenen Verfahren bisher nicht realisiert werden.

Aufgabe der vorliegenden Erfindung ist daher die Bereitstellung eines neuen Verfahrens zur Herstellung von elektronenarmen Biphenylen, insbesondere von solchen, die mit mehreren (2,_3 oder 4) Halogenatomen substituiert sind, das die Nachteile der bekannten Verfahren, vor allem die Notwendigkeit unwirtschaftlicher Synthesebausteine wie die oben erwähnten Edukte, nicht aufweist.

Diese Aufgabe wird gelöst durch ein Verfahren zum Herstellen von substituierten Biphenylen der Formel (III) 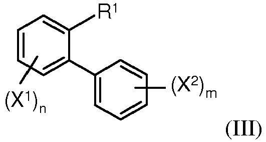 wobei
- X¹ und X²: unabhängig voneinander ausgewählt sind aus Fluor und Chlor

- n: ausgewählt ist aus 0 und 1;
- m: ausgewählt ist aus 1, 2 und 3;
- R¹: ausgewählt ist aus der Gruppe bestehend aus -NHR², -NR³-CO-R² und -N=CR⁴R⁵;
- R² - R⁵: ausgewählt sind aus der Gruppe bestehend aus Wasserstoff, linearen oder verzweigten C₁₋₆-Alkyl-Gruppen, -CH₂-CO-CH₃, Pyrazolyl-Gruppen der Formel (IVa) und Pyridyl-Gruppen der Formel (IVb) 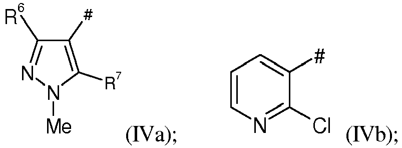
wobei in den Formeln (IVa) und (IVb), wenn R¹ für -NR³-CO-R² steht, jeweils die mit # markierte Bindung mit der Carbonyl-Gruppe verbunden ist;
R⁶ und R⁷ ausgewählt sind aus Wasserstoff, Halogen, einer linearen oder verzweigten C₁₋₁₂-Alkyl-Gruppe und einer C₁₋₆-Haloalkyl-Gruppe mit 1 bis 6 Halogenatomen;
durch Umsetzen von Arenen der Formel (I) 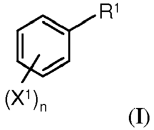 wobei
R¹, X¹ und n den obigen Definitionen entsprechen,
mit Arenen der Formel (II) 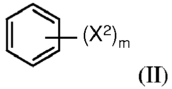 wobei
- X² und m: den obigen Definitionen entsprechen,
wobei die Reaktion in Gegenwart eines Übergangsmetallkatalysators, mindestens eines Oxidationsmittels, bis zu zehn, bevorzugt bis zu fünf, besonders bevorzugt bis zu drei Additiven und in einem Lösungsmittel durchgeführt wird.

Diese Lösung der erfinderischen Aufgabe ist überraschend gegenüber dem Stand der Technik. So wird zwar in Joanna Wencel-Delord et al., Angew. Chem. Int. Ed. 2012, 51, 2247-2251 eine Rh-katalysierte dehydrogenierende Aryl-Aryl-Kupplung beschrieben, die den Einsatz elektronenarmer Aromaten erlaubt. Diese Umsetzungen durch C-H-Aktivierung wurden bisher allerdings nur mit Benzamiden als dirigierendem Kupplungspartner durchgeführt. Die in der vorliegenden Erfindung als Edukte verwendeten Arene der Formel (I) weisen gegenüber Benzamiden eine stark unterschiedliche elektronische Situation auf. Von einem vergleichbar guten ortho-dirigierenden Effekt wie er bei der Reaktion von Benzamiden auftritt und nicht mit dem +/- I bzw. +/- M-Effekt bei der nucleophilen aromatischen Substitution zu verwechseln ist, kann bei den im erfindungsgemäßen Verfahren als Edukte verwendeten Arenen der Formel (I) nicht notwendigerweise ausgegangen werden. Typische Beispiele von ortho-dirigierenden Gruppen sind bei Eric J.-G. Anctil und Victor Snieckus in Metal-Catalyzed Cross-Coupling Reactions, 2nd Edition 2004, 761-813. Hrsg. A. de Meijere und F. Diederich, WILEY-VCH Verlag GmbH & KGaA, Weinheim (Schema 14-2, Seite 762) zu finden. Hier werden Benzamide explizit als ortho-dirigierende Gruppen genannt, nicht aber die entsprechenden Anilide.

Im Zusammenhang mit der vorliegenden Erfindung umfasst der Begriff Halogene, soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden.

Gegebenenfalls substituierte Gruppen können einfach oder mehrfach substituiert sein, wobei bei Mehrfachsubstitutionen die Substituenten gleich oder verschieden sein können.

Die mit einem oder mehreren Halogenatomen substituierten Alkyl-Gruppen sind beispielsweise ausgewählt aus Trifluormethyl (-CF₃), Difluormethyl (-CHF₂), CH₂CF₃, CH₂Cl, und CCl₂CF₃.

Alkyl-Gruppen sind im Zusammenhang mit der vorliegenden Erfindung, soweit nicht abweichend definiert, lineare, oder verzweigte Kohlenwasserstoff-Gruppen, die optional ein, zwei oder mehrere Heteroatome, die ausgewählt sind aus O, N, P und S aufweisen können. Außerdem können die erfindungsgemäßen Alkyl-Gruppen optional durch weitere Gruppen substituiert sein, die ausgewählt sind aus-R', Halogen-, Alkoxy- (-OR'), Thioether- oder Mercapto- (-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₁-C₁₂-Alkyl umfasst den größten hierin definierten Bereich für eine Alkyl-Gruppe. Im Einzelnen umfasst diese Definition beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Cycloalkyl-Gruppen sind im Zusammenhang mit der vorliegenden Erfindung, soweit nicht abweichend definiert, ringförmige Kohlenwasserstoff-Gruppen, die optional ein, zwei oder mehrere Heteroatome, die ausgewählt sind aus O, N, P und S aufweisen können. Außerdem können die erfindungsgemäßen Cycloalkyl-Gruppen optional durch weitere Gruppen substituiert sein, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₃-C₈-Cycloalkyl umfasst den größten hierin definierten Bereich für eine CycloalkylGruppe. Im Einzelnen umfasst diese Definition beispielsweise die Bedeutungen Cyclopropyl, Cyclobutyl, Cyclohexyl, Cycloheptyl und Cyclooctyl.

Aryl-Gruppen sind im Zusammenhang mit der vorliegenden Erfindung, soweit nicht abweichend definiert, aromatische Kohlenwasserstoff-Gruppen, die ein, zwei oder mehrere Heteroatome, die ausgewählt sind aus O, N, P und S aufweisen können und optional durch weitere Gruppen substituiert sein können, die ausgewählt sind aus -R', Halogen-, Alkoxy- (-OR'), Thioether- oder Mercapto- (-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₅₋₁₈-Aryl umfasst den größten hierin definierten Bereich für eine Aryl-Gruppe mit 5 bis 18 Gerüst-Atomen, wobei die C-Atome gegen Heteroatome ausgetauscht sein können. Im Einzelnen umfasst diese Definition beispielsweise die Bedeutungen Cyclopentadienyl, Phenyl, Cycloheptatrienyl, Cyclooctatetraenyl, Naphthyl und Anthracenyl; 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5- Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl; 1-Pyrrolyl, 1-Pyrazolyl, 1,2,4-Triazol-1-yl, 1-Imidazolyl, 1,2,3-Triazol-1-yl, 1,3,4-Triazol-1-yl; 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

Arylalkyl-Gruppen (Aralkyl-Gruppen) sind im Zusammenhang mit der vorliegenden Erfindung, soweit nicht abweichend definiert, durch Aryl-Gruppen substituierte Alkyl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen-, Alkoxy- (-OR'), Thioether- oder Mercapto-(-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₇₋₁₉-Aralkyl-Gruppe umfasst den größten hierin definierten Bereich für eine Arylalkyl-Gruppe mit insgesamt 7 bis 19 Atomen in Gerüst und Alkylenkette. Im Einzelnen umfasst diese Definition beispielsweise die Bedeutungen Benzyl- und Phenylethyl-.

Alkylaryl-Gruppen (Alkaryl-Gruppen) sind im Zusammenhang mit der vorliegenden Erfindung, soweit nicht abweichend definiert, durch Alkyl-Gruppen substituierte Aryl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen-, Alkoxy- (-OR'), Thioether- oder Mercapto-(-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₇₋₁₉-Alkaryl-Gruppe umfasst den größten hierin definierten Bereich für eine AlkylarylGruppe mit insgesamt 7 bis 19 Atomen in Gerüst und Alkylenkette. Im Einzelnen umfasst diese Definition beispielsweise die Bedeutungen Tolyl-, 2,3-, 2,4-, 2,5-, 2,6-, 3,4- oder 3,5-Dimethylphenyl.

Die Alkyl-, Alkenyl-, Alkinyl-, Aryl,- Alkaryl- und Aralkylgruppen können zudem ein oder mehrere Heteroatome aufweisen, die - soweit nicht abweichend definiert - ausgewählt sind aus N, O, P und S. Die Heteroatome ersetzen dabei die bezifferten Kohlenstoffatome. Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart und beansprucht.

Gemäß einer bevorzugten Ausführungsform des erfindungsgemäßen Verfahrens zum Herstellen von substituierten Biphenylen der Formel (III) sind die Substituenten wie folgt definiert:
- X¹ und X²: sind unabhängig voneinander ausgewählt aus Fluor und Chlor;

- n: ist ausgewählt aus 0 und 1;
- m: ist ausgewählt aus 1, 2 und 3;
- R¹: ist ausgewählt aus -NHR², -NR³-CO-R² und -N=CR⁴R⁵;
- R²-R⁵: sind ausgewählt aus Wasserstoff, linearen oder verzweigten C₁₋₆-Alkyl-Gruppen, - CH₂-CO-CH₃, Pyrazolyl-Gruppen der Formel (IVa) und Pyridyl-Gruppen der Formel (IVb) 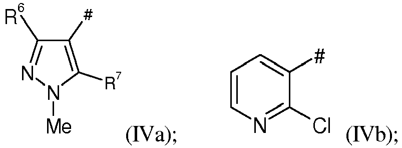
wobei in den Formeln (IVa) und (IVb), wenn R¹ für -NR³-CO-R² steht, jeweils die mit # markierte Bindung mit der Carbonyl-Gruppe verbunden ist;
R⁶ und R⁷ sind ausgewählt aus Wasserstoff, Halogen, einer linearen oder verzweigten C₁₋₆-Alkyl-Gruppe und einer C₁₋₆-Haloalkyl-Gruppe mit 1 bis 6 Halogenatomen.

Gemäß einer weiteren bevorzugten Ausführungsform des erfindungsgemäßen Verfahrens zum Herstellen von substituierten Biphenylen der Formel (III) sind die Substituenten wie folgt definiert:
- X¹ und X²: sind unabhängig voneinander ausgewählt aus Fluor und Chlor;

- n: ist ausgewählt aus 0 und 1;
- m: ist ausgewählt aus 1, 2 und 3;
- R¹: ist ausgewählt aus -NHR² und -NR³-CO-R²;
- R²-R⁵: sind ausgewählt aus Wasserstoff, linearen oder verzweigten C₁₋₆-Alkyl-Gruppen, -CH₃-CO-CH₃, Pyrazolyl-Gruppen der Formel (IVa) und Pyridyl-Gruppen der Formel (IVb) 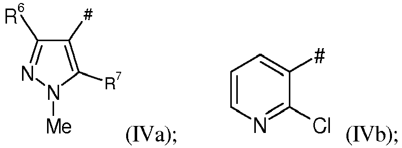
wobei in den Formeln (IVa) und (IVb), wenn R¹ für -NR³-CO-R² steht, jeweils die mit # markierte Bindung mit der Carbonyl-Gruppe verbunden ist;
R⁶ und R⁷ sind ausgewählt aus Wasserstoff, Halogen, einer linearen oder verzweigten C₁₋₆-Alkyl-Gruppe und einer C₁₋₆-Haloalkyl-Gruppe mit 1 bis 6 Halogenatomen.

Gemäß einer weiteren bevorzugten Ausführungsform des erfindungsgemäßen Verfahrens zum Herstellen von substituierten Biphenylen der Formel (III) sind die Substituenten wie folgt definiert:
- X¹ und X²: sind unabhängig voneinander ausgewählt aus Fluor und Chlor;

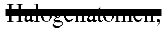

- n: ist ausgewählt aus 0 und 1;
- m: ist ausgewählt aus 1, 2 und 3;
- R¹: ist -NR³-CO-R²;
- R² - R⁵: sind ausgewählt aus Wasserstoff, linearen oder verzweigten C₁₋₆-Alkyl-Gruppen, -CH₃-CO-CH₃, Pyrazolyl-Gruppen der Formel (IVa) und Pyridyl-Gruppen der Formel (IVb) 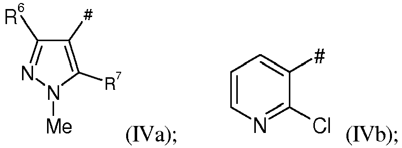
wobei in den Formeln (IVa) und (IVb), wenn R¹ für -NR³-CO-R² steht, jeweils die mit # markierte Bindung mit der Carbonyl-Gruppe verbunden ist;
R⁶ und R⁷ sind ausgewählt aus Wasserstoff, Halogen, einer linearen oder verzweigten C₁₋₆-Alkyl-Gruppe und einer C₁₋₆-Haloalkyl-Gruppe mit 1 bis 6 Halogenatomen.

Gemäß einer besonders bevorzugten Ausführungsform des erfindungsgemäßen Verfahrens zum Herstellen von substituierten Biphenylen der Formel (III) sind die Substituenten in Formel (III) wie folgt definiert:
- X¹: ist 5-Fluor;
- n: ist 1;
- X²: ist 3,4-Chlor;
- m: ist 2;
- R¹: ist ausgewählt aus -NH₂ und -NH-CO-R²;
- R²: ist ausgewählt aus Methyl, - CH₂-CO-CH₃ und Pyrazolyl-Gruppen der Formel (IVa) 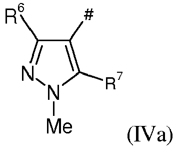
wobei in der Formel (IVa), wenn R¹ für -NH-CO-R² steht, jeweils die mit # markierte Bindung mit der Carbonyl-Gruppe verbunden ist;
- R⁶: ist Difluormethyl und
- R⁷: ist Wasserstoff.

Gemäß einer ganz besonders bevorzugten Ausführungsform des erfindungsgemäßen Verfahrens zum Herstellen von substituierten Biphenylen der Formel (III) sind die Substituenten in Formel (III) wie folgt definiert:
- X¹: ist 5-Fluor;
- n: ist 1;
- X²: ist 3,4-Chlor;
- m: ist 2;
- R¹: ist -NH-CO-R²;
- R²: ist ausgewählt aus Methyl, - CH₂-CO-CH₃ und Pyrazolyl-Gruppen der Formel (IVa) 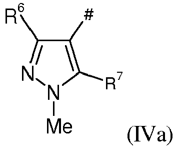
wobei in der Formel (IVa), wenn R¹ für -NH-CO-R² steht, jeweils die mit # markierte Bindung mit der Carbonyl-Gruppe verbunden ist;
- R⁶: ist Difluormethyl und
- R⁷: ist Wasserstoff.

Gemäß einer weiteren besonders bevorzugten Ausführungsform des erfindungsgemäßen Verfahrens zum Herstellen von substituierten Biphenylen der Formel (III) sind die Substituenten in Formel (III) wie folgt definiert:
- n: ist 0;
- X²: ist 3,4,5-Fluor;
- m: ist 3;
- R¹: ist ausgewählt aus -NH₂ und -NH-CO-R²;
- R²: ist ausgewählt aus Methyl, - CH₂-CO-CH₃ und Pyrazolyl-Gruppen der Formel (IVa) 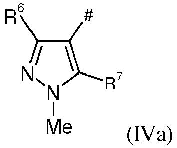
wobei in der Formel (IVa), wenn R¹ für -NH-CO-R² steht, die mit # markierte Bindung mit der Carbonyl-Gruppe verbunden ist;
- R⁶: ist Difluormethyl und
- R⁷: ist Wasserstoff.

Gemäß einer weiteren besonders bevorzugten Ausführungsform des erfindungsgemäßen Verfahrens zum Herstellen von substituierten Biphenylen der Formel (III) sind die Substituenten in Formel (III) wie folgt definiert:
- n: ist 0;
- X²: ist 3,4,5-Fluor;
- m: ist 3;
- R¹: ist -NH-CO-R²;
- R²: ist ausgewählt aus Methyl, - CH₂-CO-CH₃ und Pyrazolyl-Gruppen der Formel (IVa) 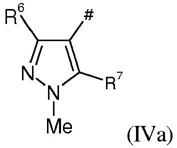
wobei in der Formel (IVa), wenn R¹ für -NH-CO-R² steht, die mit # markierte Bindung mit der Carbonyl-Gruppe verbunden ist;
- R⁶: ist Difluormethyl und
- R⁷: ist Wasserstoff.

Gemäß einer weiteren besonders bevorzugten Ausführungsform des erfindungsgemäßen Verfahrens zum Herstellen von substituierten Biphenylen der Formel (III) sind die Substituenten in Formel (III) wie folgt definiert:
- n: ist 0;
- X²: ist 4-Chlor;
- m: ist 1;
- R¹: ist ausgewählt aus -NH₂ und -NH-CO-R²;
- R²: ist ausgewählt aus Methyl, - CH₂-CO-CH₃ und Pyridyl-Gruppen der Formel (IVb) 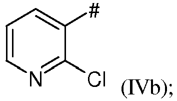
wobei in der Formel (IVb), wenn R¹ für -NH-CO-R² steht, die mit # markierte Bindung mit der Carbonyl-Gruppe verbunden ist.

Gemäß einer weiteren besonders bevorzugten Ausführungsform des erfindungsgemäßen Verfahrens zum Herstellen von substituierten Biphenylen der Formel (III) sind die Substituenten in Formel (III) wie folgt definiert:
- n: ist 0;
- X²: ist 4-Chlor;
- m: ist 1;
- R¹: ist -NH-CO-R²;
- R²: ist ausgewählt aus Methyl, - CH₂-CO-CH₃ und Pyridyl-Gruppen der Formel (IVb) 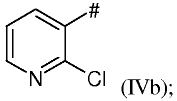
wobei in der Formel (IVb), wenn R¹ für -NH-CO-R² steht, die mit # markierte Bindung mit der Carbonyl-Gruppe verbunden ist.

Die Arene der Formel (I) sind im Zusammenhang mit der vorliegenden Erfindung wie folgt substituiert. 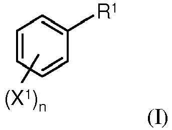
- X¹: ist unabhängig voneinander ausgewählt aus Fluor und Chlor;

- n: ist ausgewählt aus 0 und 1;
- R¹: ist ausgewählt aus -NHR², -NR³-CO-R² und -N=CR⁴R⁵;
- R² - R⁵: sind ausgewählt aus Wasserstoff, linearen oder verzweigten C₁₋₁₂-Alkyl-Gruppen, cyclischen C₃₋₈-Alkyl-Gruppen, - CH₂-CO-CH₃, Benzyl-Gruppen, Benzoyl-Gruppen, Pyrazolyl-Gruppen der Formel (IVa) und Pyridyl-Gruppen der Formel (IVb) 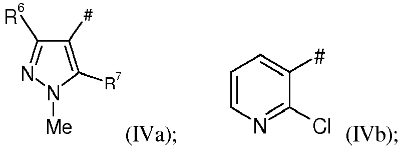
wobei in den Formeln (IVa) und (IVb), wenn R¹ für -NR³-CO-R² steht, jeweils die mit # markierte Bindung mit der Carbonyl-Gruppe verbunden ist;
R⁶ und R⁷ sind ausgewählt aus Wasserstoff, Halogen, einer linearen oder verzweigten C₁₋₁₂-Alkyl-Gruppe und einer C₁₋₆-Haloalkyl-Gruppe mit 1 bis 6 Halogenatomen;

Gemäß einer bevorzugten Ausführungsform der vorliegenden Erfindung sind die Arene der Formel (I) wie folgt substituiert. 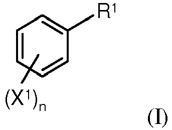
- X¹: ist unabhängig voneinander ausgewählt aus Halogenatomen;
- n: ist ausgewählt aus 0 und 1;
- R¹: ist ausgewählt aus -NHR², -NO₂, -NR³-CO-R² und -N=CR⁴R⁵;
- R² - R⁵: sind ausgewählt aus Wasserstoff, linearen oder verzweigten C₁₋₆-Alkyl-Gruppen, -CH₂-CO-CH₃, Pyrazolyl-Gruppen der Formel (IVa) und Pyridyl-Gruppen der Formel (IVb) 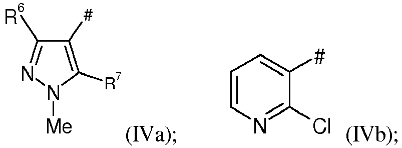
wobei in den Formeln (IVa) und (IVb), wenn R¹ für -NR³-CO-R² steht, jeweils die mit # markierte Bindung mit der Carbonyl-Gruppe verbunden ist;
R⁶ und R⁷ sind ausgewählt aus Wasserstoff, Halogen, einer linearen oder verzweigten C₁₋₁₂-Alkyl-Gruppe und einer C₁₋₆-Haloalkyl-Gruppe mit 1 bis 6 Halogenatomen;

Gemäß einer bevorzugten Ausführungsform der vorliegenden Erfindung sind die Arene der Formel (I) wie folgt substituiert. 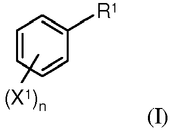
- X¹: ist 4-Fluor;
- n: ist ausgewählt aus 0 und 1;
- R¹: ist ausgewählt aus -NHR² und -NR³-CO-R²;
R² - R⁵ sind ausgewählt aus Wasserstoff, linearen oder verzweigten C₁₋₆-Alkyl-Gruppen,-CH₂-CO-CH₃, Pyrazolyl-Gruppen der Formel (IVa) und Pyridyl-Gruppen der Formel (IVb) 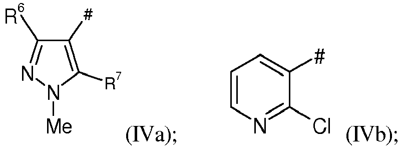 wobei in den Formeln (IVa) und (IVb), wenn R¹ für -NR³-CO-R² steht, jeweils die mit # markierte Bindung mit der Carbonyl-Gruppe verbunden ist;
R⁶ und R⁷ sind ausgewählt aus Wasserstoff, Halogen, einer linearen oder verzweigten C₁₋₁₂-Alkyl-Gruppe und einer C₁₋₆-Haloalkyl-Gruppe mit 1 bis 6 Halogenatomen.

Gemäß einer bevorzugten Ausführungsform der vorliegenden Erfindung sind die Arene der Formel (I) wie folgt substituiert. 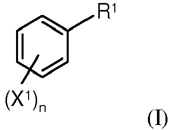
- X¹: ist 4-Fluor;
- n: ist ausgewählt aus 0 und 1;
- R¹: ist -NR³-CO-R²;
R² - R⁵ sind ausgewählt aus Wasserstoff, linearen oder verzweigten C₁₋₆-Alkyl-Gruppen,-CH₂-CO-CH₃, Pyrazolyl-Gruppen der Formel (IVa) und Pyridyl-Gruppen der Formel (IVb) 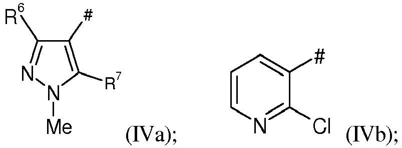 wobei in den Formeln (IVa) und (IVb), wenn R¹ für -NR³-CO-R² steht, jeweils die mit # markierte Bindung mit der Carbonyl-Gruppe verbunden ist;
R⁶ und R⁷ sind ausgewählt aus Wasserstoff, Halogen, einer linearen oder verzweigten C₁₋₁₂-Alkyl-Gruppe und einer C₁₋₆-Haloalkyl-Gruppe mit 1 bis 6 Halogenatomen.

In einer weiteren bevorzugten Ausführungsform der Erfindung sind die Arene der Formel (I) ausgewählt aus Pyrazolyl- oder Pyridyl-Aniliden (R¹ = -NH-CO-R²-), die Pyrazolyl-Gruppen der Formel (IVa) mit R⁶ = CHF₂ und R⁷ = Wasserstoff oder Fluor oder Pyridyl-Gruppen der Formel (IVb) enthalten.

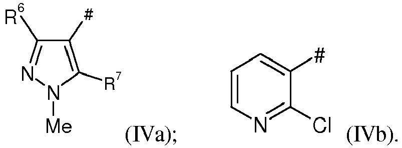

Besonders bevorzugt sind N-Phenyl-3-(difluormethyl)-1-methyl-1H-pyrazol-4-carboxamid, N-(4-Fluorphenyl)-3-(difluormethyl)-1-methyl-1H-pyrazol-4-carboxamid und N-Phenyl-2-chlornicotinamid.

Gemäß einer weiteren bevorzugten Ausführungsform des erfindungsgemäßen Verfahrens sind die Arene der Formel (I) ausgewählt aus Anilin, 4-Fluoranilin, Acetanilid, 4-Fluoracetanilid, N-Phenyl-3-oxobutanamid, N-(4-Fluorphenyl)-3-oxobutanamid, N-(Propan-2-yliden)anilin, 4-Fluor-N-(propan-2-yliden)anilin, N-Phenyl-3-(difluormethyl)-1-methyl-1H-pyrazol-4-carboxamid, N-(4-Fluorphenyl)-3-(difluormethyl)-1-methyl-1H-pyrazol-4-carboxamid und N-Phenyl-2-chlornicotinamid.

Gemäß einer besonders bevorzugten Ausführungsform des erfindungsgemäßen Verfahrens sind die Arene der Formel (I) ausgewählt aus Anilin, 4-Fluoranilin, N-(Propan-2-yliden)anilin und 4-Fluor-N-(propan-2-yliden)anilin, ganz besonders bevorzugt aus Anilin, 4-Fluoranilin.

Gemäß einer weiteren besonders bevorzugten Ausführungsform des erfindungsgemäßen Verfahrens sind die Arene der Formel (I) ausgewählt aus Acetanilid, 4-Fluoracetanilid, N-Phenyl-3-oxobutanamid, N-(4-Fluorphenyl)-3-oxobutanamid, N-Phenyl-3-(difluormethyl)-1-methyl-1H-pyrazo1-4-carboxamid, N-(4-Fluorphenyl)-3-(difluormethyl)-1-methyl-1H-pyrazol-4-carboxamid und N-Phenyl-2-chlornicotinamid, ganz besonders bevorzugt aus Acetanilid, 4-Fluoracetanilid, N-Phenyl-3-(difluormethyl)-1-methyl-1H-pyrazol-4-carboxamid, N-(4-Fluorphenyl)-3-(difluormethyl)-1-methyl-1H-pyrazol-4-carboxamid und N-Phenyl-2-chlornicotinamid.

Gemäß einer weiteren bevorzugten Ausführungsform des erfindungsgemäßen Verfahrens sind die Arene der Formel (II) ausgewählt aus 1,2-Dichlorbenzol, Chlorbenzol und 1,2,3-Trifluorbenzol.

Die Arene der Formel (II) sind im Zusammenhang mit der vorliegenden Erfindung wie folgt substituiert. 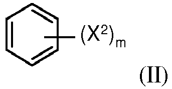
- X²: ist ausgewählt aus Fluor und Chlor;

- m: ist ausgewählt aus 1, 2 und 3.

Gemäß einer bevorzugten Ausführungsform der vorliegenden Erfindung sind die Arene der Formel (II) wie folgt substituiert. 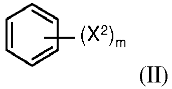
- X²: ist ausgewählt aus Fluor und Chlor;
- m: ist ausgewählt aus 1, 2 und 3.

Gemäß einer bevorzugten Ausführungsform der vorliegenden Erfindung sind die Arene der Formel (II) wie folgt substituiert. 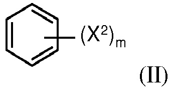
- X²: ist ausgewählt aus Chlor und Fluor;
- m: ist ausgewählt aus 1, 2 und 3, besonders bevorzugt aus 2 und 3.

Gemäß einer besonders bevorzugten Ausführungsform der vorliegenden Erfindung sind die Arene der Formel (II) ausgewählt aus 1,2-Dichlorbenzol, Chlorbenzol und 1,2,3-Trifluorbenzol.

Die Kupplung der Arene der Formel (I) mit den Arenen der Formel (II) findet vorzugsweise in Gegewart mindestens eines Lösungsmittels statt, welches beispielsweise ausgewählt ist aus der Gruppe bestehend aus Wasser, aliphatischen Ethern, ggfs. halogenierten aromatischen oder aliphatischen Kohlenwasserstoffen, Alkoholen, Estern, aromatischen oder aliphatischen Nitrilen und dipolar aprotischen Lösungsmitteln, wie Dialkylsulfoxiden, N,N-Dialkylamiden aliphatischer Carbonsäuren oder alkylierten Lactamen.

Besonders bevorzugt sind Lösungsmittel, die ausgewählt sind aus der Gruppe bestehend aus 1,2-Dichlorbenzol, Chlorbenzol, 1,2,3-Trifluorbenzol, THF, Dioxan, Diethylether, Diglyme, Methyl-tert-butylether (MTBE), tert-Amyl-methylether (TAME), Dimethylether (DME), 2-Methyl-THF, Acetonitril, Butyronitril, Toluol, Xylole, Mesitylen, Anisol, Ethylacetat, Isopropylacetat, Methanol, Ethanol, Propanol, Butanol, Ethylenglycol, Ethylencarbonat, Propylencarbonat, N,N-Dimethylacetamid, N,N-Dimethylformamid , N-Methylpyrrolidon, Wasser und Gemischen dieser.

Gemäß einer besonders bevorzugten Ausführungsform der vorliegenden Erfindung wird das Aren der Formel (II) als Lösungsmittel verwendet. Gemäß einer besonders bevorzugten Ausführungsform der vorliegenden Erfindung ist das Lösungsmittel ausgewählt aus 1,2-Dichlorbenzol, 1,2,3-Trifluorbenzol und Chlorbenzol.

Die Aryl-Aryl-Kupplung verläuft in Gegenwart von Übergangsmetallkatalysatoren. Grundsätzlich können alle im Stand der Technik im Zusammenhang mit Aryl-Aryl-Kupplungen beschriebenen Übergangsmetallkatalysatoren verwendet werden.

Vorzugsweise werden Katalysatoren auf Basis der Übergangsmetalle M eingesetzt, wobei
- M: ausgewählt ist aus den Metallen Ni, Pd, Pt, Cu, Ag, Au, Co, Rh, Ir, Fe, Ru, Mn, Cr und Ti.

Gemäß einer bevorzugten Ausführungsform ist M ausgewählt aus Ru, Pd und Rh. Gemäß einer besonders bevorzugten Ausführungsform ist M ausgewählt aus Ru und Pd. Gemäß einer ganz besonders bevorzugten Ausführungsform ist M Ru.

Die Übergangsmetalle M können in den Oxidationsstufen von -II bis +VI vorliegen.

Als Katalysatoren können auch Metallsalze bestehend aus den oben genannten Metallen M in Form von MYₚ eingesetzt werden, wobei
- M: ausgewählt ist aus Ni, Pd, Pt, Cu, Ag, Au, Co, Rh, Ir, Fe, Ru, Mn, Cr und Ti, bevorzugt aus Ru, Pd und Rh, besonders bevorzugt aus Ru und Pd, ganz besonders bevorzugt ist M Ru;
- Y: unabhängig voneinander ausgewählt ist aus F, Cl, Br, I, OTf, OAc, OMes, OTos, CF₃CO₂, SO₄ und Acetylacetonat, bevorzugt aus Cl und OAc.
- p: ausgewählt ist aus 1, 2, 3, 4, 5 und 6, bevorzugt aus 2, 3 und 4.

Als Katalysatoren können auch Übergangsmetallkomplexe der Formel MYₚLᵣ verwendet werden, die durch Kombination der oben genannten Übergangsmetallsalze MYₚ mit Liganden L erzeugt werden können, wobei
- M: ausgewählt ist aus Ni, Pd, Pt, Cu, Ag, Au, Co, Rh, Ir, Fe, Ru, Mn, Cr und Ti, bevorzugt aus Ru, Pd und Rh, besonders bevorzugt aus Ru und Pd, ganz besonders bevorzugt ist M Ru;
- Y: unabhängig voneinander ausgewählt ist aus F, Cl, Br, I, OTf, OAc, OMes, OTos, CF₃CO₂, SO₄ und Acetylacetonat, bevorzugt aus Cl und OAc;
- p: ausgewählt ist aus 1, 2, 3, 4, 5 und 6, bevorzugt aus 2, 3 und 4;
- L: unabhängig voneinander ausgewählt ist aus Cp (Cyclopentadienid), Cp*(Pentamethylcyclopentadienid), p-Cymol, PR'₃ und Phosphoramidat;
- R': unabhängig voneinander ausgewählt ist aus C₁₋₆-Alkyl-, C₃₋₅-Cycloalkyl, C₆₋₁₂-Aryl, C₁₂₋₂₄-Biaryl und Phosphinoferrocenliganden; und
- r: ausgewählt ist aus 0, 1, 2, 3, 4, 5 und 6, bevorzugt aus 2, 3 und 4.

Gemäß einer bevorzugten Ausführungsform der vorliegenden Erfindung sind die Phosphinoferrocenliganden ausgewählt aus 1,1-Bis-(di-tert-butylphosphino)ferrocen und Pentaphenyl(di-tert-butylphosphino)ferrocen.

Gemäß einer besonders bevorzugten Ausführungsform ist der Übergangsmetallkomplex der Formel MYₚLᵣ [RhCp*Cl₂]₂.

Bei der Durchführung der Reaktion kann das Katalysatorsystem (Übergangsmetall oder Übergangsmetallsalz und Ligand) zusammen oder getrennt zugegeben werden. Die Zugabe erfolgt bei Raumtemperatur oder bei einer Temperatur von 30°C bis 100°C. Bevorzugt erfolgt die Zugabe bei Raumtemperatur. Man kann den Übergangsmetallkomplexkatalysator kurz vor der Durchführung separat durch Zusammengeben eines Übergangsmetallsalzes und des Liganden herstellen oder in kristalliner Form käuflich erwerben. Bevorzugt werden die in der Reaktion verwendeten Übergangsmetallkatalysatoren *in situ* aus mindestens einem Übergangsmetallsalz MYₚ und den entsprechenden Liganden L erzeugt. Die Übergangsmetallsalze können aber auch direkt, d.h. ohne Kombination mit den Liganden L, eingesetzt werden, ohne dass dadurch die anfängliche katalytische Aktivität gemindert wird.

Gemäß einer bevorzugten Ausführungsform der vorliegenden Erfindung wird der Übergangsmetallkomplex MYₚLᵣ *in situ* aus dem Übergangsmetallsalz MYₚ und dem Liganden L erzeugt.

Gemäß einer bevorzugten Ausführungsform der vorliegenden Erfindung liegt das molare Verhältnis von Übergangsmetall M zu Ligand L zwischen 4:1 und 1:50. Gemäß einer besonders bevorzugten Ausführungsform liegt das molare Verhältnis von Übergangsmetall M zu Ligand L zwischen 1:1 und 1:5. Gemäß einer ganz besonders bevorzugten Ausführungsform liegt das molare Verhältnis von Übergangsmetall M zu Ligand L zwischen 1:1 und 1:2.

Bei dem erfindungsgemäßen Verfahren werden 0,001 bis 10,0 mol%, vorzugsweise 0,01 bis 5,0 mol%, besonders bevorzugt 0,1 bis 2,5 mol% des Übergangsmetall-Katalysators - bezogen auf das Aren der Formel (I) - eingesetzt.

Gemäß der vorliegenden Erfindung wird zur Kupplung der Arene (I) und (II) mindestens ein unabhängig voneinander kombinierbares Oxidationsmittel eingesetzt. Grundsätzlich können alle geeigneten Oxidationsmittel sowie elektrochemische Oxidation verwendet werden. Gemäß einer bevorzugten Ausführungsform sind die Oxidationsmittel ausgewählt aus Benzoquinon, Luftsauerstoff, O₂, AgNO₃, AgOAc, Cu(OAc)₂, Ag₂CO₃, AgSbF₆, K₂S₂O₈, H₄PMo₁₁VO₄₀, Cu(OTf)₂, Na₂S₂O₈. Gemäß einer besonders bevorzugten Ausführungsform wird Luftsauerstoff als Oxidationsmittel eingesetzt. Gemäß einer weiteren besonders bevorzugten Ausführungsform werden AgSbF₆ und Cu(OAc)₂ als Oxidationsmittel eingesetzt.

In Kombination mit dem Katalysator werden bei dem erfindungsgemäßen Verfahren 0,1 bis 10,0 Äquivalente, vorzugsweise 0,5 bis 5 Äquivalente, besonders bevorzugt 1,0 bis 2,5 Äquivalente des Oxidationsmittels oder der unabhängig voneinander kombinierbaren Oxidationsmittel - bezogen auf das Aren der Formel (I) - eingesetzt. Bei Einsatz von AgSbF₆ und Cu(OAc)₂ als Oxidationsmittel werden 0,05-0,5, vorzugsweise 0,05-0,2 Äquivalente AgSbF₆ und 0,5 bis 5 Äquivalente, bevorzugt 1,0 bis 2,5 Äquivalente Cu(OAc)₂ bezogen auf das Aren der Formel (I) - eingesetzt.

Gemäß der vorliegenden Erfindung können zur Kupplung der Arene der Formeln (I) und (II) bis zu zehn, bevorzugt bis zu fünf, besonders bevorzugt bis zu drei unabhängig voneinander kombinierbare Additive eingesetzt werden. Additive können zum einen Chelatoren sein, die über 1-3 Koordinationsstellen chelatisieren. Koordinationsstellen können hierbei Heteroatome und Mehrfachbindungen sein, bevorzugt O- und N-Atome sowie Doppelbindungen. Nicht-beschränkende Beispiele dafür sind sind pTsOH, AcOH, TFA, CsOPiv, PivOH. Daneben können Additive auch Hilfsmittel sein, die zur Freisetzung der katalytischen Aktivität dienen. Nicht-beschränkende Beispiele hierfür sind KPF₆, PPh₃, NH₄PF₆, NaBF₄.

Gemäß einer bevorzugten Ausführungsform der vorliegenden Erfindung sind die Additive ausgewählt aus pTsOH, AcOH, TFA, CsOPiv, PivOH, KPF₆, PPh₃, NH₄PF₆, und NaBF₄.

Gemäß einer weiteren bevorzugten Ausführungsform der vorliegenden Erfindung sind die Additive ausgewählt aus pTsOH, AcOH, TFA, CsOPiv und PivOH. Gemäß einer besonders bevorzugten Ausführungsform sind die Additive ausgewählt aus CsOPiv und PivOH.

Gemäß einer weiteren bevorzugten Ausführungsform der vorliegenden Erfindung sind die Additive ausgewählt aus KPF₆, PPh₃, NH₄PF₆, NaBF₄.

Gemäß einer bevorzugten Ausführungsform werden bis zu fünf unabhängig voneinander kombinierbare Additive eingesetzt. Gemäß einer besonders bevorzugten Ausführungsform werden bis zu drei unabhängig voneinander kombinierbare Additive eingesetzt.

Gemäß einer bevorzugten Ausführungsform der vorliegenden Erfindung werden 0,01 bis 10 Äquivalente, vorzugsweise 0,05 bis 5 Äquivalente, besonders bevorzugt 0,1 bis 1,5 Äquivalente des Additivs oder der unabhängig voneinander kombinierbaren Additive - bezogen auf das Aren der Formel (I) - eingesetzt.

Gemäß der vorliegenden Erfindung werden die Arene der Formel (I) und die Arene der Formel (II) im Verhältnis zwischen 5:1 und 1:5, vorzugsweise im Verhältnis zwischen 2:1 und 1:2 (I:II) eingesetzt. Alternativ kann jedoch auch eine der beiden Komponenten (I oder II), vorzugsweise das Aren der Formel (II), in großem Überschuss als Lösungsmittel eingesetzt werden.

Gemäß einer bevorzugten Ausführungsform der vorliegenden Erfindung wird das Aren der Formel (I) oder das Aren der Formel (II), vorzugsweise das Aren der Formel (II), in großem Überschuss als Lösungsmittel eingesetzt.

Die Umsetzung wird im allgemeinen bei einer Temperatur von 20 bis 200°C, vorzugsweise von 50 bis 150°C, besonders bevorzugt von 120 bis 140°C, sowie bei einem Druck zwischen Normaldruck und 100 bar, vorzugsweise bei einem Druck zwischen Normaldruck und 40 bar, durchgeführt.

Gemäß einer bevorzugten Ausführungsform erfolgt die Reaktion unter Ausschluss von Luftsauerstoff unter Schutzgasatmosphäre, wie z.B. unter Argon- oder Stickstoffatmosphäre.

Aufgrund der geringen Katalysatormengen kann der Katalysator in den meisten Fällen im Endprodukt verbleiben. Alternativ kann jedoch auch eine Aufreinigung der erhaltenen Biaryle durch Filtration z.B. über Celite erfolgen.

Das nachstehende Beispiel dient der Erläuterung des erfindungsmäßen Verfahrens, ohne es darauf zu beschränken:

### Synthesebeispiel:

### Beispiel 1: Kupplung von N-(4-Fluorphenyl)acetamid mit 1,2-Dichlorbenzol in Gegenwart von [RhCp*Cl₂]₂

153,2 mg (1,00 mmol) *N*-(4-Fluorphenyl)acetamid, 15,5 mg (0,02 mmol) [RhCp*Cl₂]₂, 34,4 mg (0,10 mmol) AgSbF₆, 399,6 mg (2,20 mmol) Cu(OAc)₂, 112,3 mg (1,10 mmol) PivOH und 46,8 mg (0,2 mmol) CsOPiv wurden unter Ausschluss von Sauerstoff in einem ausgeheizten Radley Reaktionsrohr vorgelegt und mit 5,0 mL (44,4 mmol) 1,2-Dichlorbenzol versetzt. Die Reaktionsmischung wurde 19 Stunden bei 130 °C gerührt. Nach Beendigung der Umsetzung (HPLC-Kontrolle) wurde die Reaktionsmischung auf RT abgekühlt, über eine kurze Kieselgelsäule filtriert und mit EtOAc eluiert. Das Lösungsmittel wurde destillativ entfernt und das Rohprodukt mittels präparativer HPLC aufgereinigt. Man erhielt 134,0 mg N-(3',4'-Dichlor-5-fluorbiphenyl-2-yl)acetamid mit einer LC-Reinheit von 99.9 % (Ausbeute 45 %).

## Patentansprüche

1. Verfahren zum Herstellen von substituierten Biphenylen der Formel (III) 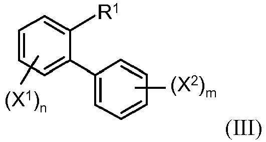 wobei
X¹ und X² unabhängig voneinander ausgewählt sind aus Fluor und Chlor;
n ausgewählt ist aus 0 und 1;
m ausgewählt ist aus 1, 2 und 3;
R¹ ausgewählt ist aus -NHR², -NR³-CO-R² und -N=CR⁴R⁵;
R² - R⁵ ausgewählt sind aus Wasserstoff, linearen oder verzweigten C₁₋₆-Alkyl-Gruppen, -CH₂-CO-CH₃, Pyrazolyl-Gruppen der Formel (IVa) und Pyridyl-Gruppen der Formel (IVb) 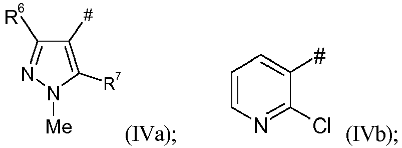 wobei in den Formeln (IVa) und (IVb), wenn R1 für -NR³-CO-R² steht, jeweils die mit # markierte Bindung mit der Carbonyl-Gruppe verbunden ist.
R⁶ und R⁷ ausgewählt sind aus Wasserstoff, Halogen, einer linearen oder verzweigten C₁₋₆-Alkyl-Gruppe und einer C₁₋₆-Haloalkyl-Gruppe mit 1 bis 6 Halogenatomen;
durch Umsetzen von Arenen der Formel (I) 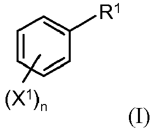 wobei
R¹, X¹ und n den obigen Definitionen entsprechen,
mit Arenen der Formel (II) 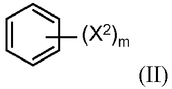 wobei
X² und m den obigen Definitionen entsprechen,
wobei die Reaktion in Gegenwart eines Übergangsmetallkatalysators, mindestens eines Oxidationsmittels, bis zu fünf Additiven und in einem Lösungsmittel durchgeführt wird.

2. Verfahren nach Anspruch 1, wobei
R¹ -NR³-CO-R² ist.

3. Verfahren nach einem der Ansprüche 1 oder 2, wobei
X¹ in Formel (III) 5-Fluor ist;
n 1 ist;
X² in Formel (III) 3,4-Chlor ist;
m 2 ist;
R¹ -NH-CO-R² ist;
R² ausgewählt ist aus Methyl, - CH₂-CO-CH₃ und Pyrazolyl-Gruppen der Formel (IVa) 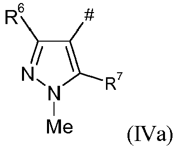 wobei in der Formel (IVa), wenn R¹ für -NH-CO-R² steht, jeweils die mit # markierte Bindung mit der Carbonyl-Gruppe verbunden ist;
R⁶ Difluormethyl ist und
R⁷ Wasserstoff ist.

4. Verfahren nach einem der Ansprüche 1 oder 2, wobei
n 0 ist;
X² in Formel (III) 3,4,5-Fluor ist;
m 3 ist;
R¹ -NH-CO-R² ist;
R² ausgewählt ist aus Methyl, - CH₂-CO-CH₃ und Pyrazolyl-Gruppen der Formel (IVa) 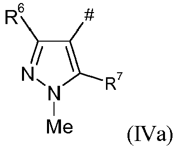 wobei in der Formel (IVa), wenn R¹ für -NH-CO-R² steht, jeweils die mit # markierte Bindung mit der Carbonyl-Gruppe verbunden ist;
R⁶ Difluormethyl ist und
R⁷ Wasserstoff ist.

5. Verfahren nach einem der Ansprüche 1 oder 2, wobei
n 0 ist;
X² in Formel (III) 4-Chlor ist;
m 1 ist;
R¹ -NH-CO-R² ist;
R² ausgewählt ist aus der Gruppe bestehend aus Methyl, - CH₂-CO-CH₃ und Pyridyl-Gruppen der Formel (IVb) 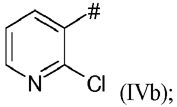 wobei in der Formel (IVb), wenn R¹ für -NH-CO-R² steht, jeweils die mit # markierte Bindung mit der Carbonyl-Gruppe verbunden ist.

6. Verfahren nach einem der Ansprüche 1 bis 5, wobei die Arene der Formel (I) ausgewählt sind aus Anilin, 4-Fluoranilin, Acetanilid, 4-Fluoracetanilid, N-Phenyl-3-oxobutanamid, N-(4-Fluorphenyl)-3-oxobutanamid, N-(Propan-2-yliden)anilin, 4-Fluor-N-(propan-2-yliden)anilin, N-Phenyl-3-(difluormethyl)-1-methyl-1H-pyrazol-4-carboxamid, N-(4-Fluorphenyl)-3-(difluormethyl)-1-methyl-1H-pyrazol-4-carboxamid und N-Phenyl-2-chlornicotinamid.

7. Verfahren nach einem der Ansprüche 1 bis 6, wobei die Arene der Formel (II) ausgewählt sind aus 1,2-Dichlorbenzol, Chlorbenzol und 1,2,3-Trifluorbenzol.

8. Verfahren nach einem der Ansprüche 1 bis 7, wobei das Lösungsmittel ausgewählt ist aus 1,2-Dichlorbenzol, Chlorbenzol, 1,2,3-Trifluorbenzol, THF, Dioxan, Diethylether, Diglyme, Methyl-tert-butylether (MTBE), tert-Amyl-methylether (TAME), Dimethylether (DME), 2-Methyl-THF, Acetonitril, Butyronitril, Toluol, Xylole, Mesitylen, Anisol, Ethylacetat, Isopropylacetat, Methanol, Ethanol, Propanol, Butanol, Ethylenglycol, Ethylencarbonat, Propylencarbonat, N,N-Dimethylacetamid, N,N-Dimethylformamid, N-Methylpyrrolidon, Wasser und Gemischen dieser.

9. Verfahren nach einem der Ansprüche 1 bis 8, wobei der Übergangsmetallkatalysator ausgewählt ist aus den Übergangsmetallen M, wobei M ausgewählt ist aus Ni, Pd, Pt, Cu, Ag, Au, Co, Rh, Ir, Fe, Ru, Mn, Cr und Ti.

10. Verfahren nach einem der Ansprüche 1 bis 8, wobei der Übergangsmetallkatalysator ausgewählt ist aus den Übergangsmetallsalzen MYₚ, wobei
M ausgewählt ist aus Ni, Pd, Pt, Cu, Ag, Au, Co, Rh, Ir, Fe, Ru, Mn, Cr und Ti;
Y unabhängig voneinander ausgewählt ist aus F, Cl, Br, I, OTf, OAc, OMes, OTos, CF₃CO₂, SO₄ und Acetylacetonat;
p ausgewählt ist aus 1, 2, 3, 4, 5 und 6.

11. Verfahren nach einem der Ansprüche 1 bis 8, wobei der Übergangsmetallkatalysator ausgewählt ist aus den Übergangsmetallkomplexen MYₚLᵣ, wobei
M ausgewählt ist aus Ni, Pd, Pt, Cu, Ag, Au, Co, Rh, Ir, Fe, Ru, Mn, Cr und Ti;
Y unabhängig voneinander ausgewählt ist aus F, Cl, Br, I, OTf, OAc, OMes, OTos, CF₃CO₂, SO₄ und Acetylacetonate;
p ausgewählt ist aus 1, 2, 3, 4, 5 und 6;
L unabhängig voneinander ausgewählt ist aus Cp (Cyclopentadienid), Cp*(Pentamethylcyclopentadienid), p-Cymol, PR'₃ und Phosphoramidat;
R' unabhängig voneinander ausgewählt ist aus C₁₋₆-Alkyl-, C₃₋₅-Cycloalkyl, C₆₋₁₂-Aryl, C₁₂₋₂₄-Biaryl und Phosphinoferrocenliganden; und
r ausgewählt ist aus 0, 1, 2, 3, 4, 5 und 6.

12. Verfahren nach einem der Ansprüche 1 bis 11, wobei das mindestens eine Oxidationsmittel ausgewählt ist aus Benzoquinon, Luftsauerstoff, O₂, AgNO₃, AgOAc, Cu(OAc)₂, Ag₂CO₃, AgSbF₆, K₂S₂O₈, H₄PMo₁₁VO₄₀, Cu(OTf)₂ und Na₂S₂O₈.

13. Verfahren nach einem der Ansprüche 1 bis 12, wobei die bis zu fünf Additive ausgewählt sind aus pTsOH, AcOH, TFA, CsOPiv, PivOH, KPF₆, PPh₃, NH₄PF₆ und NaBF₄.

14. Verfahren nach einem der Ansprüche 1 bis 13, wobei das Aren der Formel (I) oder das Aren der Formel (II), vorzugsweise das Aren der Formel (II), in großem Überschuss als Lösungsmittel eingesetzt wird.

## Claims

1. Process for preparing substituted biphenyls of the formula (III) 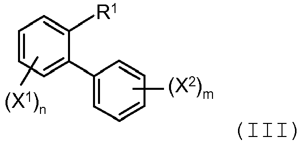 where
X¹ and X² are each independently selected from fluorine and chlorine;
n is selected from 0 and 1;
m is selected from 1, 2 and 3;
R¹ is selected from -NHR², -NR³-CO-R² and -N=CR⁴R⁵;
R² - R⁵ are each selected from hydrogen, linear or branched C₁₋₆-alkyl groups, -CH₂-CO-CH₃, pyrazolyl groups of the formula (IVa) and pyridyl groups of the formula (IVb) 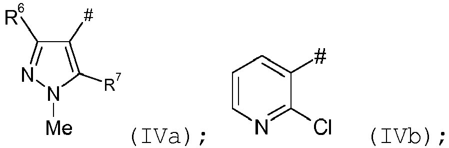
where, in the formulae (IVa) and (IVb), when R¹ is -NR³-CO-R², the bond marked with # is bonded to the carbonyl group in each case,
R⁶ and R⁷ are each selected from hydrogen, halogen, a linear or branched C₁₋₆-alkyl group and a C₁₋₆-haloalkyl group having 1 to 6 halogen atoms;
by reacting arenes of the formula (I) 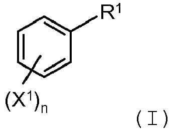 where
R¹, X¹ and n correspond to the above definitions with arenes of the formula (II) 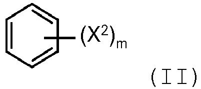 where
X² and m correspond to the above definitions, the reaction being performed in the presence of a transition metal catalyst, of at least one oxidizing agent, up to five additives and in a solvent.

2. Process according to Claim 1, wherein
R¹ is -NR³-CO-R².

3. R¹ Process according to either of Claims 1 and 2, wherein
X¹ in formula (III) is 5-fluoro;
n is 1;
X² in formula (III) is 3,4-chloro;
m is 2;
R¹ is -NH-CO-R²;
R² is selected from methyl, - CH₂-CO-CH₃ and pyrazolyl groups of the formula (IVa) 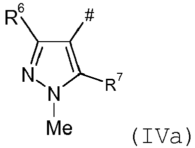 where, in the formula (IVa), when R¹ is -NH-CO-R², the bond marked with # is bonded to the carbonyl group in each case;
R⁶ is difluoromethyl and
R⁷ is hydrogen.

4. Process according to either of Claims 1 and 2, wherein
n is 0;
X² in formula (III) is 3,4,5-fluoro;
m is 3;
R¹ is -NH-CO-R²;
R² is selected from methyl, - CH₂-CO-CH₃ and pyrazolyl groups of the formula (IVa) 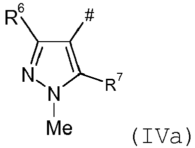 where, in the formula (IVa), when R¹ is -NH-CO-R², the bond marked with # is bonded to the carbonyl group in each case;
R⁶ is difluoromethyl and
R⁷ is hydrogen.

5. Process according to either of Claims 1 and 2, wherein
n is 0;
X² in formula (III) is 4-chloro;
m is 1;
R¹ is -NH-CO-R²;
R² is selected from the group consisting of methyl, - CH₂-CO-CH₃ and pyridyl groups of the formula (IVb) 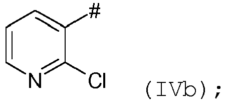 where, in the formula (IVb), when R¹ is -NH-CO-R², the bond marked with # is bonded to the carbonyl group in each case.

6. Process according to any of Claims 1 to 5, wherein the arenes of the formula (I) are selected from aniline, 4-fluoroaniline, acetanilide, 4-fluoroacetanilide, N-phenyl-3-oxobutanamide, N-(4-fluorophenyl)-3-oxobutanamide, N-(propan-2-ylidene)aniline, 4-fluoro-N-(propan-2-ylidene)aniline, N-phenyl-3-(difluoromethyl)-1-methyl-1H-pyrazole-4-carboxamide, N-(4-fluorophenyl)-3-(difluoromethyl)-1-methyl-1H-pyrazole-4-carboxamide and N-phenyl-2-chloronicotinamide.

7. Process according to any of Claims 1 to 6, wherein the arenes of the formula (II) are selected from 1,2-dichlorobenzene, chlorobenzene and 1,2,3-trifluorobenzene.

8. Process according to any of Claims 1 to 7, wherein the solvent is selected from 1,2-dichlorobenzene, chlorobenzene, 1,2,3-trifluorobenzene, THF, dioxane, diethyl ether, diglyme, methyl tert-butyl ether (MTBE), tert-amyl methyl ether (TAME), dimethyl ether (DME), 2-methyl-THF, acetonitrile, butyronitrile, toluene, xylenes, mesitylene, anisole, ethyl acetate, isopropyl acetate, methanol, ethanol, propanol, butanol, ethylene glycol, ethylene carbonate, propylene carbonate, N,N-dimethylacetamide, N,N-dimethylformamide, N-methylpyrrolidone, water and mixtures of these.

9. Process according to any of Claims 1 to 8, wherein the transition metal catalyst is selected from the transition metals M, M being selected from Ni, Pd, Pt, Cu, Ag, Au, Co, Rh, Ir, Fe, Ru, Mn, Cr and Ti.

10. Process according to any of Claims 1 to 8, wherein the transition metal catalyst is selected from the transition metal salts MYₚ where
M is selected from Ni, Pd, Pt, Cu, Ag, Au, Co, Rh, Ir, Fe, Ru, Mn, Cr and Ti;
Y is independently selected from F, Cl, Br, I, OTf, OAc, OMes, OTos, CF₃CO₂, SO₄ and acetylacetonate;
p is selected from 1, 2, 3, 4, 5 and 6.

11. Process according to any of Claims 1 to 8, wherein the transition metal catalyst is selected from the transition metal complexes MYₚLᵣ where
M is selected from Ni, Pd, Pt, Cu, Ag, Au, Co,
Rh, Ir, Fe, Ru, Mn, Cr and Ti;
Y is independently selected from F, Cl, Br, I, OTf, OAc, OMes, OTos, CF₃CO₂, SO₄ and acetylacetonates;
p is selected from 1, 2, 3, 4, 5 and 6;
L is independently selected from Cp (cyclopentadienide), Cp*(pentamethylcyclopentadienide), p-cymene, PR'₃ and phosphoramidate;
R' is independently selected from C₁₋₆-alkyl, C₃₋₅-cycloalkyl, C₆₋₁₂-aryl, C₁₂₋₂₄-biaryl and phosphinoferrocene ligands; and
r is selected from 0, 1, 2, 3, 4, 5 and 6.

12. Process according to any of Claims 1 to 11, wherein the at least one oxidizing agent is selected from benzoquinone, atmospheric oxygen, O₂, AgNO₃, AgOAc, Cu(OAc)₂, Ag₂CO₃, AgSbF₆, K₂S₂O₈, H₄PMo₁₁VO₄₀, Cu(OTf)₂ and Na₂S₂O₈.

13. Process according to any of Claims 1 to 12, wherein the up to five additives are selected from pTsOH, AcOH, TFA, CsOPiv, PivOH, KPF₆, PPh₃, NH₄PF₆ and NaBF₄.

14. Process according to any of Claims 1 to 13, wherein the arene of the formula (I) or the arene of the formula (II), preferably the arene of the formula (II), is used in a large excess as a solvent.

## Revendications

1. Procédé de fabrication de biphényles substitués de formule (III) 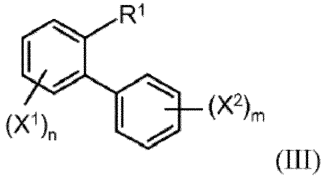 dans laquelle
X¹ et X² sont choisis indépendamment l'un de l'autre parmi fluor et chlore ;
n est choisi parmi 0 et 1 ;
m est choisi parmi 1, 2 et 3 ;
R¹ est choisi parmi -NHR², -NR³-CO-R² et -N=CR⁴R⁵ ;
R² à R⁵ sont choisis parmi l'hydrogène, les groupes alkyle en C₁₋₆ linéaires ou ramifiés, -CH₂-CO-CH₃, les groupes pyrazolyle de formule (IVa) et les groupes pyridyle de formule (IVb) 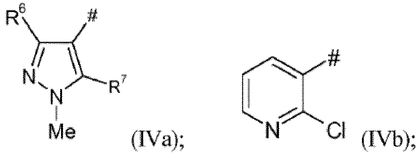
dans les formules (IVa) et (IVb), lorsque R1 représente -NR³-CO-R², la liaison marquée avec # étant à chaque fois reliée avec le groupe carbonyle,
R⁶ et R⁷ sont choisis parmi l'hydrogène, un halogène, un groupe alkyle en C₁₋₆ linéaire ou ramifié et un groupe haloalkyle en C₁₋₆ contenant 1 à 6 atomes d'halogène ; par mise en réaction d'arènes de formule (I) 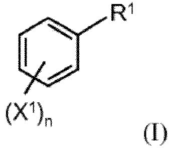 dans laquelle
R¹, X¹ et n correspondent aux définitions précédentes,
avec des arènes de formule (II) 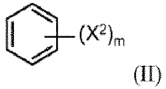 dans laquelle
X² et m correspondent aux définitions précédentes,
la réaction étant réalisée en présence d'un catalyseur de métal de transition, d'au moins un oxydant, de jusqu'à cinq additifs et dans un solvant.

2. Procédé selon la revendication 1, dans lequel R¹ représente -NR³-CO-R².

3. Procédé selon l'une quelconque des revendications 1 ou 2, dans lequel
X¹ dans la formule (III) représente 5-fluor ;
n représente 1 ;
X² dans la formule (III) représente 3,4-chlore ;
m représente 2 ;
R¹ représente -NH-CO-R² ;
R² est choisi parmi méthyle, -CH₂-CO-CH₃ et les groupes pyrazolyle de formule (IVa) 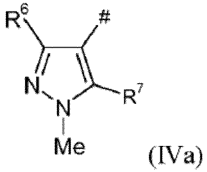 dans la formule (IVa), lorsque R¹ représente -NH-CO-R², la liaison marquée avec # étant à chaque fois reliée avec le groupe carbonyle ;
R⁶ représente difluorométhyle ; et
R⁷ représente hydrogène.

4. Procédé selon l'une quelconque des revendications 1 ou 2, dans lequel
n représente 0 ;
X² dans la formule (III) représente 3,4,5-fluor ;
m représente 3 ;
R¹ représente -NH-CO-R² ;
R² est choisi parmi méthyle, -CH₂-CO-CH₃ et les groupes pyrazolyle de formule (IVa) 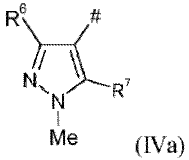 dans la formule (IVa), lorsque R¹ représente -NH-CO-R², la liaison marquée avec # étant à chaque fois reliée avec le groupe carbonyle ;
R⁶ représente difluorométhyle ; et
R⁷ représente hydrogène.

5. Procédé selon l'une quelconque des revendications 1 ou 2, dans lequel
n représente 0 ;
X² dans la formule (III) représente 4-chlore ;
m représente 1 ;
R¹ représente -NH-CO-R² ;
R² est choisi dans le groupe constitué par méthyle, -CH₂-CO-CH₃ et les groupes pyridyle de formule (IVb) 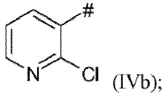 dans la formule (IVb), lorsque R¹ représente -NH-CO-R², la liaison marquée avec # étant à chaque fois reliée avec le groupe carbonyle.

6. Procédé selon l'une quelconque des revendications 1 à 5, dans lequel les arènes de formule (I) sont choisis parmi l'aniline, la 4-fluoroaniline, l'acétanilide, le 4-fluoroacétanilide, le N-phényl-3-oxobutanamide, le N-(4-fluorophényl)-3-oxobutanamide, la N-(propan-2-ylidène)aniline, la 4-fluoro-N-(propan-2-ylidène)aniline, le N-phényl-3-(difluorométhyl)-1-méthyl-1H-pyrazole-4-carboxamide, le N-(4-fluorophényl)-3-(difluorométhyl)-1-méthyl-1H-pyrazole-4-carboxamide et le N-phényl-2-chloronicotinamide.

7. Procédé selon l'une quelconque des revendications 1 à 6, dans lequel les arènes de formule (II) sont choisis parmi le 1,2-dichlorobenzène, le chlorobenzène et le 1,2,3-trifluorobenzène.

8. Procédé selon l'une quelconque des revendications 1 à 7, dans lequel le solvant est choisi parmi le 1,2-dichlorobenzène, le chlorobenzène, le 1,2,3-trifluorobenzène, le THF, le dioxane, l'éther diéthylique, le diglyme, l'éther méthyl-tert-butylique (MTBE), l'éther tert-amyl-méthylique (TAME), l'éther diméthylique (DME), le 2-méthyl-THF, l'acétonitrile, le butyronitrile, le toluène, les xylènes, le mésitylène, l'anisole, l'acétate d'éthyle, l'acétate d'isopropyle, le méthanol, l'éthanol, le propanol, le butanol, l'éthylène glycol, le carbonate d'éthylène, le carbonate de propylène, le N,N-diméthylacétamide, le N,N-diméthylformamide, la N-méthylpyrrolidone, l'eau et les mélanges de ceux-ci.

9. Procédé selon l'une quelconque des revendications 1 à 8, dans lequel le catalyseur de métal de transition est choisi parmi les métaux de transition M, M étant choisi parmi Ni, Pd, Pt, Cu, Ag, Au, Co, Rh, Ir, Fe, Ru, Mn, Cr et Ti.

10. Procédé selon l'une quelconque des revendications 1 à 8, dans lequel le catalyseur de métal de transition est choisi parmi les sels de métaux de transition MYₚ, M étant choisi parmi Ni, Pd, Pt, Cu, Ag, Au, Co, Rh, Ir, Fe, Ru, Mn, Cr et Ti ;
les Y étant choisis indépendamment les uns des autres parmi F, Cl, Br, I, OTf, OAc, OMes, OTos, CF₃CO₂, SO₄ et l'acétonate d'acétyle ;
p étant choisi parmi 1, 2, 3, 4, 5 et 6.

11. Procédé selon l'une quelconque des revendications 1 à 8, dans lequel le catalyseur de métal de transition est choisi parmi les complexes de métaux de transition MYₚLᵣ,
M étant choisi parmi Ni, Pd, Pt, Cu, Ag, Au, Co, Rh, Ir, Fe, Ru, Mn, Cr et Ti ;
les Y étant choisis indépendamment les uns des autres parmi F, Cl, Br, I, OTf, OAc, OMes, OTos, CF₃CO₂, SO₄ et l'acétonate d'acétyle ;
p étant choisi parmi 1, 2, 3, 4, 5 et 6 ;
les L étant choisis indépendamment les uns des autres parmi Cp (cyclopentadiénide), Cp*(pentaméthylcyclopentadiénide), p-cymène, PR'₃ et le phosphoramidate ;
les R' étant choisis indépendamment les uns des autres parmi alkyle en C₁₋₆, cycloalkyle en C₃₋₅, aryle en C₆₋₁₂, biaryle en C₁₂₋₂₄ et les ligands phosphinoferrocène ; et r étant choisi parmi 0, 1, 2, 3, 4, 5 et 6.

12. Procédé selon l'une quelconque des revendications 1 à 11, dans lequel ledit au moins un oxydant est choisi parmi la benzoquinone, l'oxygène de l'air, O₂, AgNO₃, AgOAc, Cu(OAc)₂, Ag₂CO₃, AgSbF₆, K₂S₂O₈, H₄PMo₁₁VO₄₀, Cu(OTf)₂ et Na₂S₂O₈.

13. Procédé selon l'une quelconque des revendications 1 à 12, dans lequel lesdits jusqu'à cinq additifs sont choisis parmi pTsOH, AcOH, TFA, CsOPiv, PivOH, KPF₆, PPh₃, NH₄PF₆ et NaBF₄.

14. Procédé selon l'une quelconque des revendications 1 à 13, dans lequel l'arène de formule (I) ou l'arène de formule (II), de préférence l'arène de formule (II), est utilisé en un excès important en tant que solvant.
